# EUROPÄISCHE PATENTSCHRIFT

(11) **EP 0 858 790 B1**
(45) Veröffentlichungstag und Bekanntmachung des Hinweises auf die Patenterteilung: **10.10.2001**
(21) Anmeldenummer: 98102219.7
(22) Anmeldetag: 09.02.1998
(51) Int. Cl.: A61F 13/00, A61F 13/36, A61F 13/15

(54) **Medizinischer Saugkörper**
Absorbing substance for medical application
Corps absorbant à usage médical

(30) Priorität: 14.02.1997 DE 19705737
(43) Veröffentlichungstag der Anmeldung: 19.08.1998
(73) Patentinhaber: Spinnerei C.B. Göldner GmbH & Co.KG, 08412 Werdau (DE)
(72) Erfinder: Goeser, Hans Joachim, 08412 Werdau (DE); Gründig, Egon, 08468 Reichenbach (DE)
(74) Vertreter: Schieschke, Klaus, Dipl.-Ing.

(56) Entgegenhaltungen:
- DE-A- 4 330 824
- GB-A- 1 138 040
- GB-A- 2 267 511
- US-A- 3 695 270
- US-A- 5 475 904

## Beschreibung

Die Erfindung bezieht sich auf einen medizinischen Saugkörper aus 100 % Baumwollfasern.

Medizinsche Saugkörper dienen der Aufnahme und dem Abtransport von Körperflüssigkeit. Sie zählen zu den sogenannten Stapelverbandstoffen und müssen physiologisch unbedenklich, sicher und einfach in der Handhabung sowie kostengünstig sein.

Im medizinischen Saugkörper sind zunächst lose Fasern, vorzugsweise Naturfasern, entweder durch vorausgegangene Fadenbildung mit anschließendem Weben, durch Wirrlage der Fasern, durch Steppen oder durch entsprechendes Umhüllungsmaterial so zusammengehalten, dass die Einzelfasern am Auseinderdriften gehindert sind. Erst mit diesem Effekt wird die Flüssigkeitsaufnahme und das Speichervermögen gewährleistet, welche ein mehrfaches des Eigengewichts des Saugkörpers ausmachen sollten. Nur wenn die Einzelfasern im Saugkörper sicher zusammengehalten werden, kann die aufgesaugte Körperflüssigkeit abgenommen bzw. entnommen und abtransportiert werden.

Sind die Fasern nur unzureichend untereinander verbunden, lässt sich der Saugkörper nicht im Gesamten entfernen und damit auch nicht die von ihm aufgesogene Flüssigkeit. Die sich verselbständigenden Fasern und Fasertrümmer verbleiben im Körper und können zu gefährlichen Abwehrreaktionen führen.

Die Saugfähigkeit eines Saugkörpers ist dann maximal, wenn alle Faserspitzen gleich hoch und parallel ausgerichtet sind. Speichervolumen und Haltevermögen hängen vom sogenannten Kapillareffekt ab, d.h. von der Entfernung der parallelliegenden Fasern untereinander, der Affinität der Fasern zum Sekret und dessen Konsistenz. Die Fähigkeit der Fasern, Flüssigkeit zu binden, sinkt, je mehr und je größer die Faseroberfläche durch Verbundstellen mit anderen Fasern belegt ist. Jede Entwicklung eines optimalen Saugkörpers muss daher die zum Teil sich widersprechenden Anforderungen, nämlich hohe Fasereinbindung und hohes Flüssigkeitssaug- und haltevermögen, optimal kombinieren. Die zum Stand der Technik zählenden, an sich bekannten Verfahren zur Herstellung von medizinischen Saugkörpern sind die Bildung von Geweben oder Gewirken aus Baumwollgarnen, die Herstellung von Vliesen aller Art aus Baumwolle, Viskose oder Chemiefasern sowie gesteppte oder umhüllte Faserflore bzw. Faserlagen.

Als Stand der Technik ist darüber hinaus beispielsweise ein Wundschnellverband bekannt (DE-PS 667 940), bei welchem ein auf einer Seite mit einem Haftmittel versehener Träger Anwendung findet, auf welchem eine elastisch ausgebildete Wundauflage aus einem Gewebe, Gewirke oder Gestricke angeordnet ist. Der Träger besteht hierbei aus einem porösen Wirrfaservlies.

Um das Auflösevermögen loser Faserverbindungen zu reduzieren, und zwar bei Erhaltung des maximalen Flüssigkeitsaufnahme- und -haltevermögens, zählt es darüber hinaus zum Stand der Technik, Kombinationen zwischen festen und losen Verbindungen zu wählen, beispielsweise Watte mit Vliesstoff oder Gewebeumhüllung oder Sauglagen mit Gewebe- und Vliesabdeckungen. Der Nachteil dieser Verbundlagen ist deren unzureichende Verknüpfung untereinander bzw. ihre relativ aufwendige Herstellung sowie bei chemischer Verbindung der Lagen untereinander deren mangelnde physiologische Unbedenklichkeit.

Entsprechend liegt der vorliegenden Erfindung die Aufgabe zugrunde, einen medizinischen Saugkörper sowie ein Verfahren zur Herstellung desselben zu schaffen, bei welchem das hergestellte Produkt ein hohes Flüssigkeitssaug- und -haltevermögen bei hoher Fasereinbindung besitzt.

Diese Aufgabe wird erfindungsgemäß bei einem medizinischen Saugkörper aus 100 % Baumwollfasern gelöst durch eine ober- und unterseitige Deckschicht der Baumwollfasern durch Vermaschung, wobei diese unter Bildung eines Zwischenraums etwa senkrecht zu der Vermaschung ausgerichtet sind. Die Maschen und die Baumwollfasern können entsprechend der erforderlichen Festigkeit kombiniert sein. Darüber hinaus können die Baumwollfasern entsprechend der Konsistenz und der Menge dicht und in etwa parallel ausgerichtet sein.

In weiterer Ausgestaltung der Erfindung besteht die Möglichkeit, dass mindestens eine vermaschte Deckschicht eine Einlage aufweist, welche beispielsweise ein Mullgewebe sein kann.

Das Verfahren zum Herstellen eines medizinischen Saugkörpers aus 100 % Baumwollfasern sieht vor, dass die Baumwollfasern direkt einseitig zur Bildung einer unteren Deckschicht vermascht werden, dass anschließend die zunächst noch offene Oberseite der Baumwollfasern zur Bildung einer oberen Deckschicht vermascht wird und dass zwischen den Maschenlagen der oberen und der unteren Deckschicht ein Zwischenraum verbleibt, in welchem die Baumwollfasern weitgehend senkrecht zu den Vermaschungen ausgerichtet sind.

Erfindungsgemäß wird also vorteilhafterweise ein vermaschtes Fasergebilde als kostengünstiger Faserverbund geschaffen, bei welchem das Verhältnis der Festigkeit des Faserverbundes und die Flüssigkeitsaufnahme optimal kombiniert sind. Hierbei werden die 100 % Baumwollfasern mit einer flüssigkeitsdurchlässigen oder auch -undurchlässigen Deckschicht fest vermascht. Es werden in dem Fasergebilde nur die Faserenden fest eingebunden. Diese sind entweder Maschenstäbchen oder Querverbindungen zwischen den Maschenstäbchen. Der Hauptteil der Baumwollfasern verbleibt senkrecht und parallel zueinander angeordnet und steht damit seiner Funktion, Flüssigkeit zu halten, voll zur Verfügung.

Vorteilhafterweise kann sich der Faserverbund bei seiner Anwendung nicht mehr in seine Bestandteile auflösen. Die Faserverbindungen werden mechanisch, d.h. ohne chemische Zusätze, und damit physiologisch unbedenklich herbeigeführt; die Baumwollfasern stehen - wie vorstehend ausgeführt - dicht und relativ parallel zueinander. Mit diesem Fasergebilde, d.h. dem neuen medizinischen Saugkörper wird die Flüssigkeitsaufnahmefähigkeit und das Flüssigkeitshaltevermögen in Relation zum eingebrachten Fasergewicht vorteilhafterweise maximiert.

Die Erfindung wird nachfolgend anhand eines in der Zeichnung dargestellten Ausführungsbeispiels näher beschrieben. In der Zeichnung zeigen:

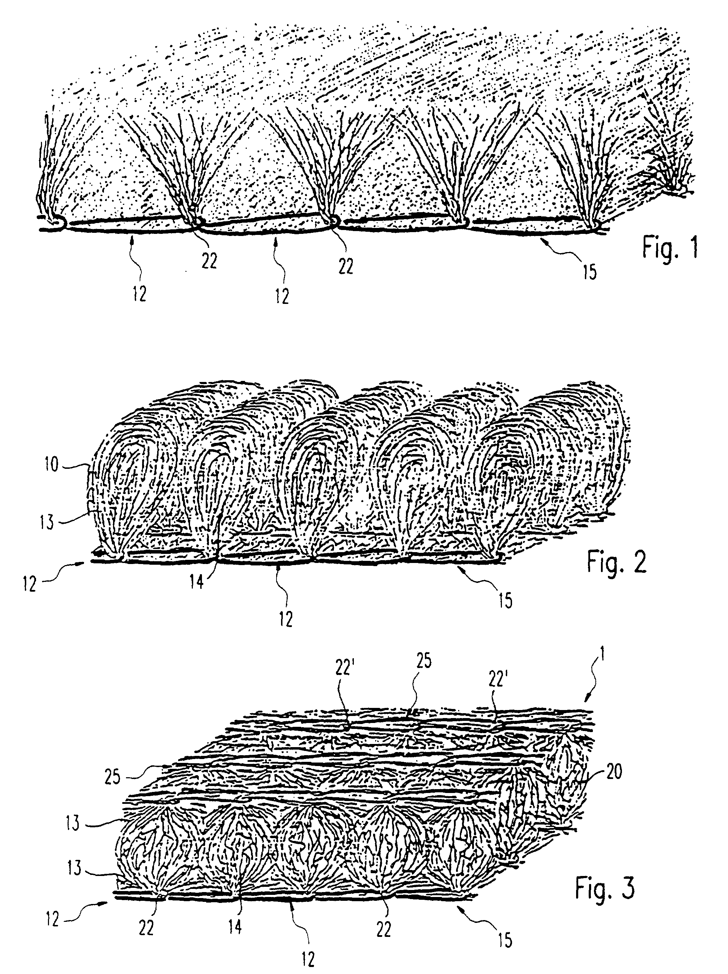

- Fig. 1 bis 3: verschiedene Verfahrensschritte zur Herstellung eines erfindungsgemäßen medizinischen Saugkörpers in schematischer Ansicht.

Gemäß Fig. 1 liegen gebündelte, 100-prozentige Baumwollfasern 10 vor, welche zur Bildung einer unteren Deckschicht 15 mit einer Vermaschung 12 versehen werden. Es liegen damit Maschen 22 vor. Die Baumwollfasern 10 werden hierbei jedoch nur so weit eingebunden, dass nach Fig. 2 noch ein großer Bestandteil für ihre eigentliche Funktion als Saugbereich 13, in welchem Flüssigkeit gebunden wird, verbleibt.

Anschließend wird die zunächst noch offene Oberseite der Baumwollfasern 10 zur Bildung einer oberen Deckschicht 25 gemäß Fig. 3 vermascht. Es liegen damit Maschen 22' vor. Wie ersichtlich, verbleibt zwischen der oberen Deckschicht 25 und der unteren Deckschicht 15 ein Zwischenraum 20, in welchem die Baumwollfasern 10 weitgehend in senkrechter Ausrichtung 14 zu den Vermaschungen 12 ausgerichtet sind. Diese Ausrichtung ist den Wirrlagen eines Vlieses oder eines Watteflors überlegen, weil durch diese Ausbildung das Flüssigkeitsspeicher- und-haltevermögen im Saugbereich 13 in Bezug auf die eingesetzte Menge der Baumwollfasern erheblich höher liegt. Andererseits liegt infolge der vermaschten Deckschichten 15 und 25 ein fester Faserverbund vor.

Die Baumwollfasern 10 werden zur Flüssigkeitsaufnahme je nach ihrer Konsistenz und Menge optimal dicht und parallel ausgerichtet; die Maschen 22 und die Baumwollfasern 10 werden je nach erforderlicher Festigkeit des Saugkörpers 1 optimal kombiniert.

Zur Herstellung eines medizinischen Saugkörpers gemäß der vorliegenden Erfindung lassen sich an sich bekannte Verfahren verwenden, wie beispielsweise ein Verfahren zur Herstellung eines großvolumigen Vliesstoffes mit einerseits verfestigten Oberflächen (DE 43 09 990 A1). Dieses bekannte Verfahren ist jedoch bis zum jetzigen Zeitpunkt lediglich zur Herstellung von Vliesstoff für Lärmdämmungen, Wärmedämmstoffe, Filterzwecke und für Polsterkombinationen eingesetzt worden. Nach einer weiteren bekannten Technologie (DE 42 18 234 C2) wird ein Vliesstoff hergestellt, welcher bisher für Bodenbeläge, Decken oder für Isolierstoffe angewendet wurde.

Insgesamt wird ein medizinischer Saugkörper geschaffen, welcher bei hohem Flüssigkeitssaug- und -haltevermögen eine hohe Fasereinbindung aufweist, ohne dass zusätzliche Umhüllungen oder Bindemittel eingesetzt werden müssen.

## Patentansprüche

1. Medizinischer Saugkörper aus 100 % Baumwollfasern,
**gekennzeichnet durch**
eine ober- und unterseitige Deckschicht (15, 25) der Baumwollfasern (10) **durch** Vermaschung (12), wobei diese Baumwollfasern unter Bildung eines Zwischenraums (20) in etwa senkrecht (14) zu der Vermaschung (12) ausgerichtet sind.

2. Saugkörper nach Anspruch 1,
**dadurch gekennzeichnet,**
**dass** die Maschen (12') und Baumwollfasern (10) entsprechend der erforderlichen Festigkeit kombiniert sind.

3. Saugkörper nach Anspruch 1 oder 2,
**dadurch gekennzeichnet,**
**dass** die Baumwollfasern (10) entsprechend der Konsistenz und der Menge dicht und in etwa parallel ausgerichtet sind.

4. Saugkörper nach einem oder mehreren der vorhergehenden Ansprüche,
**dadurch gekennzeichnet,**
**dass** mindestens eine vermaschte Deckschicht eine Einlage aufweist.

5. Saugkörper nach Anspruch 4,
**dadurch gekennzeichnet,**
**dass** die Einlage ein Mullgewebe ist.

6. Verfahren zum Herstellen eines medizinischen Saugkörpers aus 100 % Baumwollfasern,
**dadurch gekennzeichnet,**
**dass** die Baumwollfasern (10) direkt einseitig zur Bildung einer unteren Deckschicht (15) vermascht werden, dass anschließend die zunächst noch offene Oberseite der Baumwollfasern (10) zur Bildung einer oberen Deckschicht (25) vermascht wird und
**dass** zwischen den Maschen (12') der oberen und unteren Deckschicht (15, 25) ein Zwischenraum (20) verbleibt, in welchem die Baumwollfasern (10) weitgehend senkrecht (14) zu den Vermaschungen (12) ausgerichtet sind.

7. Verfahren nach Anspruch 6,
**dadurch gekennzeichnet,**
**dass** die Baumwollfasern (10) je nach Konsistenz und Menge optimal dicht und parallel ausgerichtet sind.

8. Verfahren nach Anspruch 6 und 7,
**dadurch gekennzeichnet,**
**dass** die Maschen (22, 22') der Vermaschungen (12) und die Baumwollfasern (10) je nach erforderlicher Festigkeit des Saugkörpers (1) optimal kombiniert sind.

## Claims

1. Medical absorption material of 100 % cotton fibres,
**characterized by**
a top and bottom outer layer (15, 25) of the cotton fibres (10), formed by intermeshing (12), these cotton fibres being aligned approximately perpendicularly (14) to the intermeshing (12) and an interspace (20) being formed.

2. Absorption material according to Claim 1,
**characterized in that**
the meshes (12') and cotton fibres (10) are combined according to the required strength.

3. Absorption material according to either of Claims 1 or 2,
**characterized in that**
the cotton fibres (10) are aligned closely and approximately parallel according to the consistency.

4. Absorption material according to one or more of the preceding Claims,
**characterized in that**
at least one intermeshed outer layer comprises an inlay.

5. Absorption material according to Claim 4,
**characterized in that**
the inlay is a woven mull fabric.

6. Method for producing a medical absorption material of 100 % cotton fibres,
**characterized in that**
the cotton fibres (10) are directly intermeshed on one side to form a lower outer layer (15),
the top side of the cotton fibres (10), which at first is still open, is then intermeshed to form an upper outer layer (25) and
there remains between the meshes (12') of the upper and lower outer layer (15, 25) an interspace (20) in which the cotton fibres (10) are aligned substantially perpendicularly (14) to the intermeshings (12).

7. Method according to Claim 6,
**characterized in that**
the closeness and parallelism of alignment of the cotton fibres (10) are optimized according to consistency and quantity.

8. Method according to either of Claims 6 or 7,
**characterized in that**
the meshes (22, 22') of the intermeshings (12) and the cotton fibres (10) are optimally combined according to the required strength of the absorption material (1).

## Revendications

1. Corps absorbant à usage médical formé par 100 % de fibres de coton, **caractérisé par** une couche de revêtement (15, 25) des fibres de coton (10) située sur la surface supérieure et sur la face inférieure est formée par maillage (12), ces fibres de coton étant orientées pour la formation d'un espace intercalaire (20), approximativement perpendiculairement (14) au maillage (12).

2. Corps absorbant selon la revendication 1, **caractérisé en ce que** les mailles (12') et les fibres de coton (10) sont combinées en fonction de la solidité requise.

3. Corps absorbant selon la revendication 1 ou 2, **caractérisé en ce que** les fibres de coton (10) sont disposés d'une manière dense en fonction de la consistance et de la quantité et sont orientées approximativement parallèlement entre elles.

4. Corps absorbant selon une ou plusieurs des revendications précédentes, **caractérisé en ce qu'**au moins une couche de revêtement maillée comporte un insert.

5. Corps absorbant selon la revendication 4, **caractérisé en ce que** l'insert est un tissu de mousseline.

6. Procédé pour fabriquer un corps absorbant à usage médical, constitué par 100 % de fibres de coton,
**caractérisé en ce**
**qu'**on réalise le maillage des fibres de coton (10) directement d'un côté pour former une couche de revêtement inférieure (15),
qu'ensuite on réalise le maillage de la face tout d'abord encore ouverte des fibres de coton (10) pour former une couche de revêtement supérieure (25), et
qu'entre les mailles (12') des couches supérieure et inférieure de revêtement (15, 25) subsiste un espace intercalaire (20), dans lequel les fibres de coton (10) sont orientées dans une large mesure perpendiculairement (14) au maillage (12).

7. Procédé selon la revendication 6, **caractérisé en ce que** les fibres de coton (10) sont disposées avec une densité optimale en fonction de la consistance et de la quantité et en étant orientées parallèlement entre elles.

8. Procédé selon les revendications 6 et 7, **caractérisé en ce que** les mailles (22, 22') de maillages (12) et les fibres de coton (10) sont combinées d'une manière optimale en fonction de la solidité requise du corps absorbant (1).
